# EUROPEAN PATENT SPECIFICATION

(11) **EP 3 215 620 B1**
(45) Date of publication and mention of the grant of the patent: **01.04.2020**
(21) Application number: 15857185.1
(22) Date of filing: 06.11.2015
(51) Int. Cl.: C12Q 1/6806, C12N 15/10, C12Q 1/68, G01N 35/00

(54) **CHAOTROPE- AND VOLATILE-FREE METHOD FOR PURIFYING NUCLEIC ACIDS FROM PLASMA**
VERFAHREN FREI VON CHAOTROPEN UND FLÜCHTIGEN STOFFEN ZUR AUFREINIGUNG VON NUKLEINSÄUREN AUS PLASMA
MÉTHODE SANS AGENTS CHAOTROPIQUES NI COMPOSÉS VOLATILS POUR LA PURIFICATION D'ACIDES NUCLÉIQUES ISSUS DE PLASMA

(30) Priority: 07.11.2014 US 201462076795 P
(43) Date of publication of application: 13.09.2017
(73) Proprietor: The Johns Hopkins University, Baltimore, MD 21218 (US)
(72) Inventor: OSBURN, William O., Ellicott City, Maryland 21043 (US); RAY, Stuart C., Lutherville-timonium, Maryland 21093 (US)
(74) Representative: Murgitroyd & Company
(86) International application number: PCT/US2015/059403
(87) International publication number: WO 2016/073824

(56) References cited:
- EP-A1- 1 842 913
- EP-B1- 2 329 019
- WO-A1-2014/029792
- WO-A2-2006/015326
- US-A- 5 705 628
- US-A1- 2009 048 437
- US-A1- 2011 071 031
- JUNHONG MIN ET AL: "Functional integration of DNA purification and concentration into a real time micro-PCR chip", LAB ON A, ROYAL SOCIETY OF CHEMISTRY, vol. 11, no. 2, 1 January 2011 (2011-01-01), pages 259-265, XP007917469, ISSN: 1473-0197, DOI: 10.1039/C0LC00320D
- LEE M ET AL: "Isolation of total RNA from Escherichia coli using kosmotropic Hofmeister salts", ANALYTICAL BIOCHEMISTRY, ELSEVIER, AMSTERDAM, NL, vol. 381, no. 1, 1 October 2008 (2008-10-01), pages 160-162, XP023905063, ISSN: 0003-2697, DOI: 10.1016/J.AB.2008.06.015 [retrieved on 2008-06-13]
- HOURFAR, MICHAEL K. ET AL.: 'High-throughput purification of viral RNA based on novel aqueous chemistry for nucleic acid isolation' CLINICAL CHEMISTRY vol. 51, no. 7, 01 July 2005, pages 1217 - 1222, XP002403248 DOI: 10.1373/CLINCHEM.2005.048603

## Description

### BACKGROUND

The detection of nucleic acids (NA; RNA and DNA) in human blood specimens is an important component of assays for detection of a variety of acute and chronic medical conditions (e.g., pathogen infections and the like). To assess the levels of NA, the NA must first be purified from human body fluids to remove proteins and molecules that inhibit downstream NA amplification procedures. Efficient purification of NA from human specimens is an essential element in the design of next-generation viral NA silicon microchip-based diagnostic assays. The current gold standard NA purification protocol is one described by Boom et al. (Boom, 1990), which serves as the basis for the majority of commercial NA purification kits and entails binding NA to silicon dioxide in the presence of a chaotropic salt, precipitating NA onto silicon dioxide using 70% ethanol, and resuspending it in a slightly alkaline buffer or water.

The Boom protocol is limited in its applicability to next generation, silicon microchip-based assays in at least two ways. First, chaotropic salts, such as guanidinium thiocyanate, are toxic and require special handling and disposal. This limitation precludes the use of this protocol by unskilled individuals in settings outside the laboratory. Second, the use of a 70% ethanol precipitation step presents multiple challenges when using this protocol on next generation NA silicon microchip-based diagnostic assays. For example, the ethanol precipitation step can be a potent inhibitor of NA amplification assays, result in the formation of bubbles that block microfluidic channels, and is evaporation infeasible. The prohibition of Boom-based protocols in next-generation NA diagnostic assays necessitates the development of a NA purification process that is free of chaotropic salts and volatile organic chemicals.

A few alternatives have been described that attempt to overcome the limitations of Boom-based protocols. For example, Hourfar et al. (Hourfar, 2005) describes purification of viral NA from human plasma using a silica coated magnetic bead solid phase and a protocol consisting of binding in a kosmotropic salt (ammonium sulfate) at pH 4, wash step at pH 6.8 and elution by a pH 8.5 buffer at 80°C. RNases, which interfere with NA amplification assays due to degradation of RNA, are inhibited by the use of proteinase K in the binding and wash steps of the protocol. The silica coated magnetic bead solid phase used in this method is incompatible with microchip-based assays.

U.S. Patent 6,355,792 to Merck discloses purification of NA from solutions using magnetic silica beads and a protocol consisting of binding in a pH 4 buffer without any additional salts, washing with a pH 6.5 buffer and eluting in a pH 9. This protocol is not suitable for purification of NA from plasma because RNases are not inhibited or removed.

U.S. Patent 5,705,6283 to Agencourt Technologies describes the use of magnetic, polystyrene beads functionalized with -COOH groups and a "crowding" reagent in a process known as Solid Phase Reverse Immobilization to purify NA from biological fluids. In this procedure, NA are bound to the column in the presence of high concentrations of NaCl (0.5 - 5M) and polyethylene glycol (7 - 13%). Contaminants are washed off the column using a high salt buffer and eluted in a low ionic strength buffer or water.

Current methods for purifying NA from plasma involve the use of a silica-based solid phases and toxic and/or volatile buffer systems, which preclude the use of these methods in a point-of-care diagnostic test in various resource settings.

### SUMMARY

In some aspects, the presently disclosed subject matter provides a chaotrope- and volatile-free method for purifying nucleic acids, the method comprising: (a) adding a sample comprising at least one nucleic acid to a functionalized solid phase comprising a silica or polymer backbone; (b) allowing nucleic acid in the sample to bind to the functionalized solid phase in the presence of a binding buffer comprising 0.3 to 2.0 M of kosmotropic molecules, wherein the kosmotropic molecules are selected from the group consisting of ammonium sulfate, CO₃²⁻, HPO₄²⁻, glucose, trehalose, polyhydric alcohols, and proline, and wherein said binding buffer does not comprise ethanol or a chaotropic salt; (c) removing the binding buffer; (d) washing the nucleic acid bound to the functionalized solid phase with a wash buffer; and (e) eluting the bound nucleic acid from the functionalized solid phase with an elution buffer, wherein the percent recovery of the at least one nucleic acid after step (e) is about 60% or greater.

In some aspects, the presently disclosed subject matter provides a chaotrope- and volatile-free method for purifying nucleic acids, the method comprising: (a) providing a sample comprising at least one nucleic acid; (b) adding the at least one nucleic acid to a functionalized solid phase comprising a silica or polymer backbone; (c) allowing the at least one nucleic acid to bind to the functionalized solid phase comprising a silica or polymer backbone in the presence of a binding buffer comprising 0.3 to 2.0 M of kosmotropic molecules, wherein the kosmotropic molecules are selected from the group consisting of ammonium sulfate, CO₃²⁻, HPO₄²⁻ , glucose, trehalose, polyhydric alcohols, and proline, and wherein said binding buffer does not comprise ethanol or a chaotropic salt; (d) removing the binding buffer comprising the kosmotropic molecules; (e) washing the at least one nucleic acid bound to the functionalized solid phase comprising a silica or polymer backbone with a low ionic strength wash buffer; and (f) eluting the at least one nucleic acid from the functionalized solid phase comprising a silica or polymer backbone with a low ionic strength elution buffer, wherein the percent recovery of the at least one nucleic acid after step (e) is about 60% or greater.

In other aspects, the presently disclosed subject matter provides a kit comprising at least one functionalized solid phase comprising a silica or polymer backbone and a set of instructions for using the at least one functionalized solid phase to purify a nucleic acid, wherein the instructions include:
(a) adding a sample comprising at least one nucleic acid to a functionalized solid phase comprising a silica or polymer backbone;
(b) allowing nucleic acid in the sample to bind to the functionalized solid phase in the presence of a binding buffer comprising 0.3 to 2.0 M of kosmotropic molecules, wherein the kosmotropic molecules are selected from the group consisting of ammonium sulfate, CO₃²⁻, HPO₄²⁻, glucose, trehalose, polyhydric alcohols, and proline, and wherein said binding buffer does not comprise ethanol or a chaotropic salt;
(c) removing the binding buffer;
(d) washing the nucleic acid bound to the functionalized solid phase with a wash buffer; and
(e) eluting the bound nucleic acid from the functionalized solid phase with an elution buffer, wherein the percent recovery of the at least one nucleic acid after step (e) is about 60% or greater.

Certain aspects of the presently disclosed subject matter having been stated hereinabove, which are addressed in whole or in part by the presently disclosed subject matter, other aspects will become evident as the description proceeds when taken in connection with the accompanying Examples and Figure as best described herein below.

### BRIEF DESCRIPTION OF THE FIGURE

Having thus described the presently disclosed subject matter in general terms, reference will now be made to the accompanying Figure, which is not necessarily drawn to scale, and wherein:

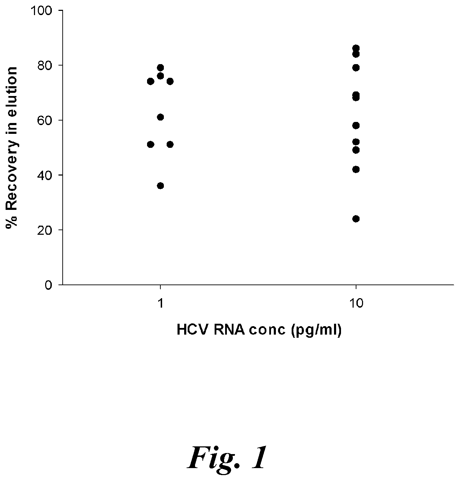

FIG. 1 shows the percent recovery of Hepatitis C virus (HCV) RNA after using the presently disclosed methods with an initial concentration of 1 and 10 pg/mL of HCV RNA.

### DETAILED DESCRIPTION

The presently disclosed subject matter now will be described more fully hereinafter with reference to the accompanying Figure, in which some, but not all embodiments of the inventions are shown. Like numbers refer to like elements throughout. The presently disclosed subject matter may be embodied in many different forms and should not be construed as limited to the embodiments set forth herein; rather, these embodiments are provided so that this disclosure will satisfy applicable legal requirements. Indeed, many modifications and other embodiments of the presently disclosed subject matter set forth herein will come to mind to one skilled in the art to which the presently disclosed subject matter pertains having the benefit of the teachings presented in the foregoing descriptions and the associated Figure. Therefore, it is to be understood that the presently disclosed subject matter is not to be limited to the specific embodiments disclosed.

The presently disclosed subject matter provides novel combinations of non-functionalized and functionalized solid phases comprising a silica or polymer backbone with non-chaotropic salt and ethanol-free buffer protocols for purifying nucleic acids. These methods can be performed on a number of different solid phases using multiple different buffer protocols that include non-chaotropic salts. In some embodiments, the methods utilize a buffer that comprises high concentrations of kosmotropic molecules, and the contaminants are removed with a low ionic strength buffer and eluted in a low ionic strength buffer. The presently disclosed subject matter overcomes the necessity of using toxic chaotropic salts and volatile organic chemicals. The presently disclosed subject matter is suitable for use in current diagnostic assays and next-generation, silicon microchip-based diagnostic assays. In addition, the presently disclosed subject matter is applicable for use in point-of-care diagnostic microfluidic or magnetic systems, thereby allowing for worldwide use of point-of-care diagnostics across all resource settings. In particular embodiments, the presently disclosed subject matter provides methods to purify nucleic acids from blood plasma.

### I. METHODS FOR PURIFYING NUCLEIC ACIDS FROM PLASMA

Accordingly, in some embodiments, the presently disclosed subject matter provides a chaotrope- and volatile-free method for purifying nucleic acids, the method comprising: (a) adding a sample comprising at least one nucleic acid to a functionalized solid phase comprising a silica or polymer backbone; (b) allowing nucleic acid in the sample to bind to the functionalized solid phase in the presence of a binding buffer comprising 0.3 to 2.0 M of kosmotropic molecules, wherein the kosmotropic molecules are selected from the group consisting of ammonium sulfate, CO₃²⁻, HPO₄²⁻, glucose, trehalose, polyhydric alcohols, and proline, and wherein said binding buffer does not comprise ethanol or a chaotropic salt; (c) removing the binding buffer; (d) washing the nucleic acid bound to the functionalized solid phase with a wash buffer; and (e) eluting the bound nucleic acid from the functionalized solid phase with an elution buffer, wherein the percent recovery of the at least one nucleic acid after step (e) is about 60% or greater.

In some embodiments, the presently disclosed subject matter provides a chaotrope- and volatile-free method for purifying nucleic acids, the method comprising: (a) providing a sample comprising at least one nucleic acid; (b) adding the at least one nucleic acid to a functionalized solid phase comprising a silica or polymer backbone; (c) allowing the at least one nucleic acid to bind to the functionalized solid phase comprising a silica or polymer backbone in the presence of a binding buffer comprising 0.3 to 2.0 M of kosmotropic molecules, wherein the kosmotropic molecules are selected from the group consisting of ammonium sulfate, CO₃²⁻, HPO₄²⁻, glucose, trehalose, polyhydric alcohols, and proline, and wherein said binding buffer does not comprise ethanol or a chaotropic salt; (d) removing the binding buffer; (e) washing the at least one nucleic acid bound to the functionalized solid phase comprising a silica or polymer backbone with a low ionic strength wash buffer; and (f) eluting the at least one nucleic acid from the functionalized solid phase comprising a silica or polymer backbone with a low ionic strength elution buffer, wherein the percent recovery of the at least one nucleic acid after step (e) is about 60% or greater.

As used herein, a "polymer backbone" refers to the series of covalently bound atoms that together create the continuous chain in a large molecule, or macromolecule, composed of many repeated subunits. As used herein, a "silica backbone" refers to a series of covalently bound silicon atoms.

As used herein, a "chaotrope" or "chaotropic agent" is an agent that disrupts water structure, increases the solubility of nonpolar solvent particles, and destabilizes solute aggregates. A "chaotrope-free" method is a method that does not use chaotropic agents. As used herein, a "kosmotropic" molecule, ionic or nonionic, causes water molecules to favorably interact and stabilizes intramolecular interactions in macromolecules, such as proteins. Ionic kosmotropes tend to be small, such as CO₃²⁻, SO₄²⁻, HPO₄²⁻, magnesium(2+), lithium(1+), zinc (2+) and aluminium (+3). As used herein, a "volatile-free" method is a method that does not use volatile molecules, such as an alcohol, and more particularly, ethanol.

As used herein, the term "sample" refers to any mixture of molecules comprising at least one nucleic acid, such as tissue of all kinds, cultured cells, body fluids, whole blood, blood serum, plasma, urine, feces, microorganisms, viruses, parasites, plants, and mixtures comprising nucleic acids following enzyme reactions. Examples of tissues include tissue from invertebrates, such as insects and mollusks, vertebrates such as fish, amphibians, reptiles, birds, and mammals such as humans, rats, dogs, cats and mice. Cultured cells can be from procaryotes, such as bacteria, blue green algae, actinomycetes, and mycoplasma and from eucaryotes, such as plants, animals, fungi, and protozoa. Blood samples include blood taken directly from an organism or blood that has been filtered in some way to remove some elements such as red blood cells, and/or serum or plasma. In some embodiments, the sample is plasma. In other embodiments, the plasma is from a human.

In some embodiments, the presently disclosed methods detect biologically relevant blood viral RNA concentrations from about 0.5 pg/mL to about 10 pg/mL, which corresponds, for example, to 1×10⁵-1×10⁶ copies of HCV RNA/mL. In other embodiments, the concentration of at least one nucleic acid in the sample is from about 0.05 pg/mL to about 1000 pg/mL. In still other embodiments, the concentration of at least one nucleic acid in the sample is from about 0.5 pg/mL to about 10 pg/mL. As used herein, a "nucleic acid" or "polynucleotide" refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms. Thus, this term includes double-stranded DNA found, inter alia, in linear or circular DNA molecules (e.g., restriction fragments), plasmids, and chromosomes. In discussing the structure of particular double-stranded DNA molecules, sequences may be described herein according to the normal convention of giving only the sequence in the 5' to 3' direction along the non-transcribed strand of DNA (i.e., the strand having a sequence homologous to the mRNA). A "recombinant DNA molecule" is a DNA molecule that has undergone a molecular biological manipulation. Non-limiting examples of RNA molecules include mRNA, tRNA, rRNA, tmRNA, miRNA, siRNA, snRNA, and dsRNA. In some embodiments, at least one nucleic acid is selected from the group consisting of RNA and DNA. In other embodiments, at least one nucleic acid is selected from the group consisting of viral, bacterial, and parasitic. Non-limiting examples of viral nucleic acids include human immunodeficiency virus (HIV) RNA, human papillomavirus (HPV) DNA, and hepatitis C virus (HCV) RNA. A non-limiting example of a parasitic nucleic acid is malaria DNA. In particular embodiments, at least one nucleic acid is selected from the group consisting of viral nucleic acid, bacterial nucleic acid, and parasitic nucleic acid. In particular embodiments, at least one nucleic acid is selected from the group consisting of RNA and DNA. In still other embodiments, the nucleic acid is RNA and the RNA is Hepatitis C virus (HCV) RNA.

As used herein, the terms "purified", "isolated" and "extracted" are used interchangeably. A "purified nucleic acid" is used herein to describe a nucleic acid that has been separated from other compounds including, but not limited to proteins, lipids, and carbohydrates. A polynucleotide is substantially pure when at least about 50%, preferably 60 to 75% of a sample exhibits a single polynucleotide sequence and conformation (linear versus covalently closed). A substantially pure polynucleotide typically comprises about 50%, preferably 60 to 90% weight/weight of a nucleic acid sample, more usually about 95%, and preferably is over about 99% pure. Polynucleotide purity or homogeneity is indicated by a number of means well known in the art, such as agarose or polyacrylamide gel electrophoresis of a sample, followed by visualizing a single polynucleotide band upon staining the gel. For certain purposes, higher resolution can be provided by using high performance liquid chromatography (HPLC) or other means well known in the art.

In some embodiments, the percent purity may not be as important as the percent recovery of a nucleic acid in methods where detection of small amounts of nucleic acid is desired. As used herein, the term "percent recovery" means the amount of nucleic acid that is found in the elution buffer after elution of the nucleic acid from the solid support compared to the initial amount found in the sample. The presently disclosed methods allow for percent recovery of more than about 60%. In some embodiments, percent recovery is greater than about 70%. In some embodiments, percent recovery is greater than about 80%. In some embodiments, percent recovery is greater than about 85%. In some embodiments, percent recovery is greater than about 90%. In other embodiments, the percent recovery of at least one nucleic acid after performing the presently disclosed methods is about 60% or greater.

In some embodiments, the presently disclosed methods provide a functionalized solid phase comprising a silica or polymer backbone for use in the purification of nucleic acids. As used herein, the term "solid phase" includes rigid and flexible solids. Non-limiting examples of solid substrates include gels, fibers, microspheres, spheres, cubes, particles of other shapes, channels, microchannels, capillaries, walls of containers, membranes and filters, beads, pillars, resin, slurry, membranes or disks, or any generally solid support material suitable for the presently disclosed methods. An advantage of using a solid phase is that it allows extensive washing to remove undesired molecules. Another advantage of using a solid phase is that it can have magnetic properties to attract nucleic acids. Still another advantage of using a solid phase is that it can be functionalized with moieties or molecules to form a "functionalized solid phase." In particular embodiments, these functionalized moieties can be used to bind nucleic acid molecules.

In some embodiments, the functionalized solid phase comprising a silica or polymer backbone has magnetic properties. In other embodiments, the functionalized solid phase comprises silica. Non-limiting examples of silica include SiO₂, amorphous silicon oxide, glass, porous silica, micromachined silicon, alkylsilica, aluminum silicate, silanized material, and oxidized silanized material. In still other embodiments, the functionalized solid phase comprising a silica or polymer backbone comprises silica beads and/or pillars. In further embodiments, the functionalized solid phase comprises at least one functional group selected from the group consisting of - COOH, -NH₃, -OH, and imidazole. In an embodiment, the functionalized solid phase comprises magnetic beads functionalized with -COOH groups.

In some embodiments, the presently disclosed methods comprise providing a sample comprising nucleic acid, adding the nucleic acid to a functionalized solid phase comprising a silica or polymer backbone, and allowing the nucleic acid to bind to the functionalized solid phase comprising a silica or polymer backbone in the presence of a binding buffer. The presently disclosed buffer systems do not utilize chaotropic salts and volatile chemicals, but instead use high concentrations of kosmotropic molecules to drive nucleic acid binding. As used herein, the term "binding" refers to two or more compounds, such as a nucleic acid and a solid phase, associating with each other in a non-covalent relationship. The kosmotropic molecules are selected from the group consisting of ammonium sulfate ((NH₄)₂SO₄), sodium chloride (NaCl), CO₃⁻², HPO₄⁻², glucose, trehalose, polyhydric alcohols, and proline. In still other embodiments, the binding of the nucleic acid to the functionalized solid phase comprising a silica or polymer backbone in the presence of binding buffer occurs for about 5 to about 10 minutes at room temperature. As used herein, the term "room temperature" refers to the typical or preferred indoor (climate-controlled) temperature to which people are generally accustomed, such as the range between 20 and 24 °C (68 and 75 °F).

As used herein, by "high concentrations of kosmotropic molecules," it is meant that the concentration of kosmotropic molecules in the binding buffer is equal to or more than about 0.3 M. For example, the concentration may be equal to or more than 0.4 M, 0.5 M, 0.6 M, 0.7 M, 0.8 M, 0.9 M, 1.0 M, 1.1 M, 1.2 M, 1.3 M, 1.4 M, 1.5 M, 1.6 M, 1.7 M, 1.8 M, or 1.9 M. The binding buffer comprises 0.3 M to 2.0 M of kosmotropic molecules. In other embodiments, the binding buffer comprises about 0.3 M to about 2.0 M ammonium sulfate. In some embodiments, the binding buffer comprises a mixture of more than one type of kosmotropic molecule, such as a combination of ammonium sulfate and sodium chloride, for example.

After allowing binding of the nucleic acid to the functionalized solid phase in the presence of binding buffer, the binding buffer is removed. Methods of removing buffer from a sample are well known in the art, such as centrifuging the sample or applying positive air pressure and/or vacuum to the sample, and therefore will not be described in detail herein.

In some embodiments, after the binding buffer is removed, the nucleic acid bound to the functionalized solid phase comprising a silica or polymer backbone is washed at least once with a low ionic strength wash buffer. In other embodiments, the low ionic strength wash buffer comprises 10 mM Tris, 10 mM MES, and the like. In still other embodiments, the low ionic strength wash buffer comprises a non-ionic detergent such as NP-40, Tween-20, Tween-80, Brij™ (Thermo Fisher Scientific, Rockford, IL), Igepal, and the like.

Accordingly, in some embodiments, the wash buffer is a low-ionic strength buffer. In other embodiments, the wash buffer comprises a non-ionic detergent. In still other embodiments, wash buffers of increasing pH are used to remove contaminants. In further embodiments, washing occurs for about 1 to about 10 minutes at room temperature. Methods of washing are well known in the art and any suitable washing scheme may be used. In still further embodiments, the binding buffer and/or the wash buffer further comprise proteinase K to remove proteins in the sample, particularly if the sample comprises plasma.

After the wash step, the nucleic acid is eluted from the functionalized solid phase comprising a silica or polymer backbone with a low ionic strength elution buffer. Non-limiting examples of low ionic strength elution buffers include 10 mM Tris, 10 mM Gly-GLy, 10 mM Bicine, and 10 mM Bis-tris propane. In some embodiments, the pH of the elution buffer is about 8 or higher. In other embodiments, the elution buffer comprises 10 mM Tris at a pH from about 8 to about 9. In still other embodiments, elution occurs at about 80°C for about 5 to about 10 minutes. In further embodiments, the purified nucleic acids can be used in amplification assays without interference from contaminating substances.

As used herein, in some embodiments, the term "low ionic strength buffer" refers to a buffer that has an ionic strength about or below 10 mM. In other embodiments, the low ionic strength buffer has an ionic strength from about 5 mM to about 10 mM. In still other embodiments, the low ionic strength buffer has an ionic strength from about 1 mM to about 10 mM. Low ionic strength buffers are well known in the art and, therefore, other low ionic strength buffers that can be used in the presently disclosed subject matter for washing or elution will not be described herein in more detail.

The presently disclosed subject matter is particularly applicable to point-of-care testing. The presently disclosed methods are simple to use, portable, and do not employ toxic or volatile buffer systems. Accordingly, in some embodiments, the method is used for point-of-care testing.

### II. KITS FOR PURIFYING NUCLEIC ACIDS

The presently disclosed subject matter also provides kits for performing the presently disclosed methods. In some embodiments, the presently disclosed subject matter provides a kit comprising at least one functionalized solid phase comprising a silica or polymer backbone and a set of instructions for using at least one functionalized solid phase comprising a silica or polymer backbone to purify a nucleic acid. Optional elements of the presently disclosed kit include suitable buffers, reagents, packaging materials, and the like. Examples of buffers or reagents include sterile water, wash buffers, and elution buffers. The reagents of the kit may be in containers in which they are stable, e.g., in lyophilized form or as stabilized liquids. Kits may supply reagents in pre-measured amounts so as to simplify the presently disclosed methods.

### III. DEFINITIONS

Although specific terms are employed herein, they are used in a generic and descriptive sense only and not for purposes of limitation. Unless otherwise defined, all technical and scientific terms used herein have the same meaning as commonly understood by one of ordinary skill in the art to which this presently described subject matter belongs.

Following long-standing patent law convention, the terms "a," "an," and "the" refer to "one or more" when used in this application, including the claims. Thus, for example, reference to "a subject" includes a plurality of subjects, unless the context clearly is to the contrary (e.g., a plurality of subjects), and so forth.

Throughout this specification and the claims, the terms "comprise," "comprises," and "comprising" are used in a non-exclusive sense, except where the context requires otherwise. Likewise, the term "include" and its grammatical variants are intended to be non-limiting, such that recitation of items in a list is not to the exclusion of other like items that can be substituted or added to the listed items.

For the purposes of this specification and appended claims, unless otherwise indicated, all numbers expressing amounts, sizes, dimensions, proportions, shapes, formulations, parameters, percentages, quantities, characteristics, and other numerical values used in the specification and claims, are to be understood as being modified in all instances by the term "about" even though the term "about" may not expressly appear with the value, amount or range. Accordingly, unless indicated to the contrary, the numerical parameters set forth in the following specification and attached claims are not and need not be exact, but may be approximate and/or larger or smaller as desired, reflecting tolerances, conversion factors, rounding off, measurement error and the like, and other factors known to those of skill in the art depending on the desired properties sought to be obtained by the presently disclosed subject matter. For example, the term "about," when referring to a value can be meant to encompass variations of, in some embodiments, ± 100% in some embodiments ± 50%, in some embodiments ± 20%, in some embodiments ± 10%, in some embodiments ± 5%, in some embodiments ±1%, in some embodiments ± 0.5%, and in some embodiments ± 0.1% from the specified amount, as such variations are appropriate to perform the disclosed methods or employ the disclosed compositions.

Further, the term "about" when used in connection with one or more numbers or numerical ranges, should be understood to refer to all such numbers, including all numbers in a range and modifies that range by extending the boundaries above and below the numerical values set forth. The recitation of numerical ranges by endpoints includes all numbers, e.g., whole integers, including fractions thereof, subsumed within that range (for example, the recitation of 1 to 5 includes 1, 2, 3, 4, and 5, as well as fractions thereof, e.g., 1.5, 2.25, 3.75, 4.1, and the like) and any range within that range.

### EXAMPLES

The following Examples have been included to provide guidance to one of ordinary skill in the art for practicing representative embodiments of the presently disclosed subject matter. In light of the present disclosure and the general level of skill in the art, those of skill can appreciate that the following Examples are intended to be exemplary only. The synthetic descriptions and specific examples that follow are only intended for the purposes of illustration, and are not to be construed as limiting in any manner to make compounds of the disclosure by other methods.

### EXAMPLE 1

### METHODS

380 µL of binding buffer (100-200 mM sodium acetate pH 4 containing from about 0.3 M to about 2.0 M ammonium sulfate and 0.5-1% NP-40) was added to 100 µL of RNA solution resulting in a final concentration of either 1 pg/mL or 10 pg/mL RNA. This solution was incubated with magnetic beads functionalized with -COOH groups for 5 to 10 minutes at room temperature. Binding buffer was then removed and the solid phase was incubated with wash buffer (10 mM Tris pH 6.5-7, 0-1% NP-40) for 1 to 10 minutes at room temperature. The wash buffer was removed and the RNA was eluted from the solid phase by incubation in 50 to 100 µL of elution buffer (10 mM Tris pH 8-9) at 80°C for 5-10 min.

### EXAMPLE 2

### RESULTS

FIG. 1 demonstrates purification efficiencies (% recovery) of HCV RNA from buffer. The mean % recovery (± std deviation) for 1 and 10 pg/mL RNA concentrations was 60% (± 20) and 62% (± 15), respectively. 1 pg HCV RNA/mL = 1×10² cp HCV RNA/µL and 10 pg HCV RNA/mL = 1×10³ cp HCV RNA/µL. These recoveries are similar to recoveries reported using other methods and allow for detection of NA across a wide spectrum of biological fluids.

### REFERENCES

All publications, patent applications, patents, and other references mentioned in the specification are indicative of the level of those skilled in the art to which the presently disclosed subject matter pertains. It will be understood that, although a number of patent applications, patents, and other references are referred to herein, such reference does not constitute an admission that any of these documents forms part of the common general knowledge in the art.
Boom R, Sol CJ, Salimans MM, Jansen CL, Wertheim-van Dillen PM, van der Noordaa J. Rapid and simple method for purification of nucleic acids. J Clin Microbiol 1990, 28(3):495-503.
Hourfar MK, Michelsen U, Schmidt M, et al. High-Throughput Purification of Viral RNA Based on Novel Aqueous Chemistry for Nucleic Acid Isolation. Clin Chem 2005, 51:1217-1222.

## Claims

1. A chaotrope- and volatile-free method for purifying nucleic acids, the method comprising:
(a) adding a sample comprising at least one nucleic acid to a functionalized solid phase comprising a silica or polymer backbone;
(b) allowing nucleic acid in the sample to bind to the functionalized solid phase in the presence of a binding buffer comprising 0.3 to 2.0 M of kosmotropic molecules, wherein the kosmotropic molecules are selected from the group consisting of ammonium sulfate, CO₃²⁻, HPO₄²⁻, glucose, trehalose, polyhydric alcohols, and proline, and wherein said binding buffer does not comprise ethanol or a chaotropic salt;
(c) removing the binding buffer;
(d) washing the nucleic acid bound to the functionalized solid phase with a wash buffer; and
(e) eluting the bound nucleic acid from the functionalized solid phase with an elution buffer,
wherein the percent recovery of the at least one nucleic acid after step (e) is about 60% or greater.

2. The method of claim 1, wherein the at least one nucleic acid is selected from the group consisting of viral nucleic acid, bacterial nucleic acid, and parasitic nucleic acid.

3. The method of claim 2, wherein the at least one nucleic acid is selected from the group consisting of RNA and DNA, wherein the RNA is optionally Hepatitis C virus (HCV) RNA.

4. The method of claim 1, wherein the sample is plasma, and wherein the plasma is optionally human plasma.

5. The method of claim 1, wherein the functionalized solid phase has magnetic properties; comprises silica beads and/or pillars; and/or comprises at least one functional group selected from the group consisting of-COOH, -NH₃, -OH, and imidazole.

6. The method of claim 1, wherein the binding buffer comprises about 0.3 M to about 2.0 M ammonium sulfate and wherein the binding buffer and/or the wash buffer optionally further comprises proteinase K.

7. The method of claim 1, wherein step (b) is performed for a period of time between about 5 minutes to about 10 minutes at room temperature; step (d) occurs for between about 1 minute to about 10 minutes at room temperature; and/or step (e) is performed at about 80 °C for a period of time between about 5 minutes to about 10 minutes.

8. The method of claim 1, wherein the concentration of the at least one nucleic acid in the sample is from about 0.05 to about 1,000 pg/mL, or optionally from about 0.5 to about 10 pg/mL.

9. The method of claim 1, wherein the wash buffer is a low ionic strength buffer or comprises a non-ionic detergent.

10. The method of claim 1, wherein the pH of the elution buffer is about 8 or higher and wherein the elution buffer optionally is a low ionic strength buffer.

11. The method of claim 1, wherein the method is used for point-of-care testing.

12. A kit comprising at least one functionalized solid phase comprising a silica or polymer backbone and a set of instructions for using the at least one functionalized solid phase comprising a silica or polymer backbone to purify a nucleic acid, wherein the instructions include:
(a) adding a sample comprising at least one nucleic acid to a functionalized solid phase comprising a silica or polymer backbone;
(b) allowing nucleic acid in the sample to bind to the functionalized solid phase in the presence of a binding buffer comprising 0.3 to 2.0 M of kosmotropic molecules, wherein the kosmotropic molecules are selected from the group consisting of ammonium sulfate, CO₃²⁻, HPO₄²⁻, glucose, trehalose, polyhydric alcohols, and proline, and wherein said binding buffer does not comprise ethanol or a chaotropic salt;
(c) removing the binding buffer;
(d) washing the nucleic acid bound to the functionalized solid phase with a wash buffer; and
(e) eluting the bound nucleic acid from the functionalized solid phase with an elution buffer, wherein the percent recovery of the at least one nucleic acid after step (e) is about 60% or greater.

## Patentansprüche

1. Ein von chaotropen und flüchtigen Stoffen freies Verfahren zum Aufreinigen von Nukleinsäuren, wobei das Verfahren Folgendes beinhaltet:
(a) Zugeben einer Probe, beinhaltend mindestens eine Nukleinsäure, zu einer funktionalisierten festen Phase, beinhaltend ein Kieselerde- oder Polymergrundgerüst;
(b) Nukleinsäure in der Probe in Gegenwart eines Bindepuffers, beinhaltend 0,3 bis 2,0 M kosmotrope Moleküle, an die funktionalisierte feste Phase binden lassen, wobei die kosmotropen Moleküle ausgewählt sind aus der Gruppe, bestehend aus Ammoniumsulfat, CO₃²⁻, HPO₄²⁻, Glucose, Trehalose, mehrwertigen Alkoholen und Prolin, und wobei der Bindepuffer kein Ethanol oder chaotropes Salz beinhaltet;
(c) Entfernen des Bindepuffers;
(d) Waschen der an die funktionalisierte feste Phase gebundenen Nukleinsäure mit einem Waschpuffer; und
(e) Eluieren der gebundenen Nukleinsäure von der funktionalisierten festen Phase mit einem Elutionspuffer,
wobei die prozentuale Rückgewinnung der mindestens einen Nukleinsäure nach Schritt (e) etwa 60 % oder mehr beträgt.

2. Verfahren gemäß Anspruch 1, wobei die mindestens eine Nukleinsäure ausgewählt ist aus der Gruppe, bestehend aus viraler Nukleinsäure, bakterieller Nukleinsäure und parasitärer Nukleinsäure.

3. Verfahren gemäß Anspruch 2, wobei die mindestens eine Nukleinsäure ausgewählt ist aus der Gruppe, bestehend aus RNA und DNA, wobei die RNA wahlweise Hepatitis-C-Virus(HCV)-RNA ist.

4. Verfahren gemäß Anspruch 1, wobei die Probe Plasma ist und wobei das Plasma wahlweise Humanplasma ist.

5. Verfahren gemäß Anspruch 1, wobei die funktionalisierte feste Phase magnetische Eigenschaften aufweist; Kieselerde-Kügelchen und/oder-säulen beinhaltet; und/oder mindestens eine funktionelle Gruppe beinhaltet, ausgewählt aus der Gruppe, bestehend aus -COOH, -NH₃, -OH und Imidazol.

6. Verfahren gemäß Anspruch 1, wobei der Bindepuffer etwa 0,3 M bis etwa 2,0 M Ammoniumsulfat beinhaltet und wobei der Bindepuffer und/oder der Waschpuffer wahlweise ferner Proteinase K beinhalten.

7. Verfahren gemäß Anspruch 1, wobei Schritt (b) über einen Zeitraum zwischen etwa 5 Minuten bis etwa 10 Minuten bei Raumtemperatur durchgeführt wird; Schritt (d) über zwischen etwa 1 Minute und etwa 10 Minuten bei Raumtemperatur stattfindet; und/oder Schritt (e) bei etwa 80 °C über einen Zeitraum zwischen etwa 5 Minuten bis etwa 10 Minuten durchgeführt wird.

8. Verfahren gemäß Anspruch 1, wobei die Konzentration der mindestens einen Nukleinsäure in der Probe etwa 0,05 bis etwa 1000 pg/ml oder wahlweise etwa 0,5 bis etwa 10 pg/ml beträgt.

9. Verfahren gemäß Anspruch 1, wobei der Waschpuffer ein Puffer mit geringer lonenstärke ist oder ein nichtionisches Detergens beinhaltet.

10. Verfahren gemäß Anspruch 1, wobei der pH-Wert des Elutionspuffers etwa 8 oder mehr beträgt und wobei der Elutionspuffer wahlweise ein Puffer mit geringer lonenstärke ist.

11. Verfahren gemäß Anspruch 1, wobei das Verfahren für patientennahes Testen verwendet wird.

12. Ein Kit, beinhaltend mindestens eine funktionalisierte feste Phase, beinhaltend ein Kieselerde- oder Polymergrundgerüst, und einen Satz Anweisungen zum Verwenden der mindestens einen funktionalisierten festen Phase, beinhaltend ein Kieselerde- oder Polymergrundgerüst, um eine Nukleinsäure aufzureinigen, wobei die Anweisungen Folgendes umfassen:
(a) Zugeben einer Probe, beinhaltend mindestens eine Nukleinsäure, zu einer funktionalisierten festen Phase, beinhaltend ein Kieselerde- oder Polymergrundgerüst;
(b) Nukleinsäure in der Probe in Gegenwart eines Bindepuffers, beinhaltend 0,3 bis 2,0 M kosmotrope Moleküle, an die funktionalisierte feste Phase binden lassen, wobei die kosmotropen Moleküle ausgewählt sind aus der Gruppe, bestehend aus Ammoniumsulfat, CO₃²⁻, HPO₄²⁻, Glucose, Trehalose, mehrwertigen Alkoholen und Prolin, und wobei der Bindepuffer kein Ethanol oder chaotropes Salz beinhaltet;
(c) Entfernen des Bindepuffers;
(d) Waschen der an die funktionalisierte feste Phase gebundenen Nukleinsäure mit einem Waschpuffer; und
(e) Eluieren der gebundenen Nukleinsäure von der funktionalisierten festen Phase mit einem Elutionspuffer, wobei die prozentuale Rückgewinnung der mindestens einen Nukleinsäure nach Schritt (e) etwa 60 % oder mehr beträgt.

## Revendications

1. Une méthode exempte de chaotropes et d'éléments volatils pour purifier des acides nucléiques, la méthode comprenant le fait :
(a) d'ajouter un échantillon comprenant au moins un acide nucléique à une phase solide fonctionnalisée comprenant un squelette de silice ou de polymère ;
(b) de permettre à de l'acide nucléique dans l'échantillon de se lier à la phase solide fonctionnalisée en présence d'un tampon de liaison comprenant de 0,3 à 2,0 M de molécules cosmotropiques, les molécules cosmotropiques étant sélectionnées dans le groupe constitué de sulfate d'ammonium, de CO₃²⁻, d'HPO₄²⁻, de glucose, de tréhalose, d'alcools polyhydriques, et de proline, et dans laquelle ledit tampon de liaison ne comprend pas d'éthanol ou un sel chaotropique ;
(c) de retirer le tampon de liaison ;
(d) de laver l'acide nucléique lié à la phase solide fonctionnalisée avec un tampon de lavage ; et
(e) d'éluer l'acide nucléique lié de la phase solide fonctionnalisée avec un tampon d'élution,
dans laquelle la récupération en pourcentage de l'au moins un acide nucléique après l'étape (e) est d'environ 60 % ou plus.

2. La méthode de la revendication 1, dans laquelle l'au moins un acide nucléique est sélectionné dans le groupe constitué d'acide nucléique viral, d'acide nucléique bactérien, et d'acide nucléique parasitaire.

3. La méthode de la revendication 2, dans laquelle l'au moins un acide nucléique est sélectionné dans le groupe constitué d'ARN et d'ADN, l'ARN étant facultativement de l'ARN du virus de l'hépatite C (VHC).

4. La méthode de la revendication 1, dans laquelle l'échantillon est du plasma, et le plasma étant facultativement du plasma humain.

5. La méthode de la revendication 1, dans laquelle la phase solide fonctionnalisée a des propriétés magnétiques ; comprend des billes et/ou des piliers de silice ; et/ou comprend au moins un groupe fonctionnel sélectionné dans le groupe constitué de -COOH, de -NH₃, d'-OH, et d'imidazole.

6. La méthode de la revendication 1, dans laquelle le tampon de liaison comprend d'environ 0,3 M à environ 2,0 M de sulfate d'ammonium et dans laquelle le tampon de liaison et/ou le tampon de lavage comprend en outre facultativement de la protéinase K.

7. La méthode de la revendication 1, dans laquelle l'étape (b) est effectuée pendant une durée allant d'entre environ 5 minutes à environ 10 minutes à température ambiante ; l'étape (d) a lieu pendant d'entre environ 1 minute à environ 10 minutes à température ambiante ; et/ou l'étape (e) est effectuée à environ 80 °C pendant une durée allant d'entre environ 5 minutes à environ 10 minutes.

8. La méthode de la revendication 1, dans laquelle la concentration de l'au moins un acide nucléique dans l'échantillon va d'environ 0,05 à environ 1 000 pg/mL, ou facultativement d'environ 0,5 à environ 10 pg/mL.

9. La méthode de la revendication 1, dans laquelle le tampon de lavage est un tampon à force ionique faible ou comprend un détergent non ionique.

10. La méthode de la revendication 1, dans laquelle le pH du tampon d'élution est d'environ 8 ou au-delà et le tampon d'élution étant facultativement un tampon à force ionique faible.

11. La méthode de la revendication 1, la méthode étant utilisée pour l'analyse hors laboratoire.

12. Une trousse comprenant au moins une phase solide fonctionnalisée comprenant un squelette de silice ou de polymère et un jeu d'instructions pour utiliser l'au moins une phase solide fonctionnalisée comprenant un squelette de silice ou de polymère afin de purifier un acide nucléique, les instructions incluant le fait :
(a) d'ajouter un échantillon comprenant au moins un acide nucléique à une phase solide fonctionnalisée comprenant un squelette de silice ou de polymère ;
(b) de permettre à de l'acide nucléique dans l'échantillon de se lier à la phase solide fonctionnalisée en présence d'un tampon de liaison comprenant de 0,3 à 2,0 M de molécules cosmotropiques, les molécules cosmotropiques étant sélectionnées dans le groupe constitué de sulfate d'ammonium, de CO₃²⁻, d'HPO₄²⁻, de glucose, de tréhalose, d'alcools polyhydriques, et de proline, et dans laquelle ledit tampon de liaison ne comprend pas d'éthanol ou un sel chaotropique ;
(c) de retirer le tampon de liaison ;
(d) de laver l'acide nucléique lié à la phase solide fonctionnalisée avec un tampon de lavage ; et
(e) d'éluer l'acide nucléique lié de la phase solide fonctionnalisée avec un tampon d'élution, dans laquelle la récupération en pourcentage de l'au moins un acide nucléique après l'étape (e) est d'environ 60 % ou plus.
